# EUROPÄISCHE PATENTANMELDUNG

(11) **EP 2 345 394 A1**
(43) Veröffentlichungstag der Anmeldung: **20.07.2011**
(21) Anmeldenummer: 10150883.6
(22) Anmeldetag: 15.01.2010
(51) Int. Cl.: A61F 9/01, G02B 26/10

(54) **Vorrichtung zum Behandeln eines Auges mit Laserstrahlung**

(71) Anmelder: Schwind eye-tech-solutions GmbH & Co. KG, 63801 Kleinostheim (DE)
(72) Erfinder: Schwed, Stefan, 64319 Pfungstad (DE); Faust, Sebastian, 63834 Sulzbach am Main (DE)
(74) Vertreter: Laub, Alexander

(57) **Zusammenfassung**

In einer Vorrichtung zum Behandeln eines Auges mit Laserstrahlung wird eine Teleskopanordnung verwendet, also werden Laserstrahlenpulse aus einer Laserquelle (12) zunächst auf eine Zwischenbildfläche (24) abgebildet, bevor sie dann auf eine Brennfläche (34) im Patientenauge (10) fokussiert werden. Dadurch können besonders kleine Lichtflecken bis hin zu 1 bis 2 µm erzeugt werden, wodurch die Präzision bei der Behandlung des Patientenauges (10) besonders hoch ist. In der Teleskopanordnung wird zur Erzeugung des Zwischenbildes bevorzugt ein F-Theta-Objektiv verwendet. Als Mittel zum Ablenken der Laserstrahlenpulse zum Zwecke des Abtastens werden bevorzugt zwei Risley-Prismen (20a, 20b) eingesetzt. Bevorzugt wird eine Mikrochip-Laserquelle (12) verwendet, die eine Wellenlänge von 355 nm abgibt.

## Beschreibung

### BESCHREIBUNG:

Die Erfindung betrifft eine Vorrichtung zum Behandeln eines Auges mit Laserstrahlung.

Die Behandlung eines Patientenauges mit Laserstrahlung basiert auf der Tatsache, dass durch Laserstrahlenpulse, welche auf die Hornhaut des Patientenauges appliziert werden, kleine Mengen an dem Hornhautgewebe zerstört werden. Die hierbei entstehenden Endprodukte verflüchtigen sich; somit kann gezielt an vorbestimmten Stellen Hornhaut des Patientenauges entfernt werden. Dies kann z. B. zum Zwecke des Einschreibens eines bestimmten Profils in die Hornhaut erfolgen, das bestimmte optische Abbildungseigenschaften bereitstellt.

Damit ein Auge mit Laserstrahlen behandelt werden kann, bedarf es einer Laserquelle zur Abgabe von Laserstrahlenpulsen. Diese werden von einer Zieleinrichtung der Vorrichtung in unterschiedliche Richtungen abgelenkt. Die Zieleinrichtung ist herkömmlicherweise aus zwei Spiegeln gebildet, welche je nach gewünschtem Zielort verkippt werden. Es gibt Ausgestaltungen, bei denen ein dritter Spiegel für eine höhere Präzision der Applikation der Laserstrahlenpulse sorgt. Aus dem US Patent 5,391,165 ist es bekannt, Risley-Prismen als Zieleinrichtung anstelle von Spiegeln zu verwenden.

Beim Betrieb der Vorrichtung gibt die Laserquelle gesteuert von einer Steuereinrichtung die Laserstrahlenpulse ab, und dieselbe Steuereinrichtung steuert die Zieleinrichtung an, um auf ganz bestimmte Stellen des Patientenauges Laserstrahlenpulse zu applizieren.

Der Zieleinrichtung sind üblicherweise Mittel zum Fokussieren der abgelenkten Laserstrahlenpulse auf das Auge nachgeordnet.

Bei herkömmlichen Vorrichtungen zum Behandeln eines Auges mit Laserstrahlung hat der Lichtfleck, den ein einzelner Laserstrahlenpuls auf der Hornhaut des Patientenauges erzeugt, typischerweise einen Durchmesser von 10 µm. Dieser Durchmesser bestimmt die Präzision, mit der ein Profil in die Hornhaut des Patientenauges eingeschrieben werden kann.

Es wäre wünschenswert, könnte die Präzision für ein solches Einschreiben eines Profils in das Patientenauge erhöht werden.

Es ist somit Aufgabe der vorliegenden Erfindung, einen Weg aufzuzeigen, wie der von einem einzelnen Laserstrahlenpuls einer Vorrichtung zum Behandeln eines Auges auf einer Hornhaut eines Patientenauges erzeugte Lichtfleck verkleinert werden kann.

Die Aufgabe wird durch eine Vorrichtung mit den Merkmalen gemäß Patentanspruch 1 gelöst.

Die Vorrichtung ist somit gekennzeichnet durch eine Teleskopanordnung. Bekanntlich umfasst eine Teleskopanordnung, dass ein erster Teil der Teleskopanordnung eine Abbildung, vorliegend der Laserstrahlenpulse, auf eine Zwischenbildfläche erzeugt, wobei vorliegend unterschiedliche Laserstrahlenpulse je nach Ablenkung durch die Zieleinrichtung auf unterschiedliche Punkte der Zwischenbildfläche abgebildet werden. Zur Teleskopanordnung gehört ferner ein zweiter Teil, der der Zwischenbildfläche im Strahlengang nachgeordnet ist, und der die Zwischenbildfläche auf Punkte einer Brennfläche abbildet.

Es ist Erkenntnis der Erfinder, dass durch das Erzeugen der Zwischenbildfläche ein Fokussieren mit einer hohen numerischen Apertur auf der Seite des Patientenauges ermöglicht ist, wobei der zweite Teil der Teleskopanordnung eine starke Fokussierung auf einen kleinen Lichtfleck ermöglicht. Eine Teleskopanordnung nimmt Raum in Anspruch; vorliegend ist dies lohnenswert, weil das Konzept des Zwischenbildes ein präzises Fokussieren ermöglicht.

Durch die Erfindung ist es möglich, mittels einer Vorrichtung zum Behandeln eines Auges mit Laserstrahlung gemäß Patentanspruch 1 Lichtflecken auf der Hornhaut des Patientenauges zu erzeugen, die einen Durchmesser von ca. 1 bis 2 µm haben, wodurch sich somit die Präzision beim Einschreiben eines Profils um das 5- bis 10-fache erhöht.

Bei einer bevorzugten Ausführungsform der Erfindung umfasst der erste Teil der Teleskopanordnung ein F-Theta-Objektiv. Während herkömmliche Objektive eine Bildhöhe proportional zum Einfallswinkel erzeugen, ist in den F-Theta-Objektiven eine Korrektur vorgesehen derart, dass die Bildhöhe proportional zum Einfallswinkel wird. Der Einfallswinkel ist im Rahmen einer Vorrichtung zum Behandeln eines Auges mit Laserstrahlung typischerweise durch die Zieleinrichtung definiert, die die einfallenden Laserstrahlenpulse unterschiedlich stark ablenkt. Die Verwendung eines F-Theta-Objektivs ermöglicht somit die Abtastung eines großes Abtastfeldes, bei der wegen der Verwendung der Teleskopanordnung gleichzeitig gegebenen hohen Präzision der Abtastung.

Bei einer bevorzugten Ausführungsform weist der zweite Teil der Teleskopanordnung eine Linse auf, die verschieblich ist, um so den Abstand der Brennfläche zu der Zwischenbildfläche zu ändern. Durch passende Einstellung der Linse können die Laserstrahlenpulse optimal in die Hornhaut eines Patientenauges appliziert werden. Durch die Verschieblichkeit der Linse kann ausgeglichen werden, wenn die Stellung des Patientenauges relativ zu der Vorrichtung von einer Sollstellung abweicht. Außerdem kann auch während der Behandlung die Linse verschoben werden, um so die Laserstrahlenpulse nach und nach mit unterschiedlicher Fokussiertiefe in der Hornhaut zu applizieren. In diesem Fall kann gegebenenfalls eine Kamera das Patientenauge beobachten und die Linse durch eine zentrale Steuereinrichtung der Vorrichtung in Abhängigkeit von Signalen der Kamera mithilfe von Steuersignalen dazu gebracht werden, sich passend zu verschieben. Hierbei kann die zentrale Steuereinrichtung z. B. ein vorbestimmtes Programm durchlaufen, damit ein Sollprofil erzielt wird, das in das Patientenauge eingeschrieben werden soll.

Bei der Vorrichtung ist bevorzugt vorgesehen, dass zwischen der Laserquelle und der Teleskopanordnung, nämlich deren erstem Teil, an sich bekannte Mittel zum Ablenken der Laserstrahlenpulse angeordnet sind, damit unterschiedlich durch diese Mittel abgelenkte Laserstrahlenpulse auf unterschiedliche Punkte der Brennfläche auftreffen.

Es hat sich gezeigt, dass viele der Vorteile, die durch die Teleskopanordnung erzielt werden, zumindest teilweise wieder verloren gehen, wenn als Mittel zum Ablenken ein Paar von Scannerspiegeln verwendet wird. Zum Zwecke des Erhalts möglichst stark fokussierter Laserstrahlenpulse (Lichtfleck mit einem Durchmesser von 1 µm bis 2 µm) ist es vorteilhaft, wenn die Mittel zum Ablenken (zwei) Risley-Prismen umfassen.

Alternativ kann zur Erzeugung eines so genannten 3-dimensionalen Scans eine Anordnung aus drei schwenkbaren Spiegeln vorgesehen sein. Hierbei kann der dritte Spiegel Fehler korrigieren, die dadurch auftreten, dass bei Auslenkung des im Strahlengang ersten Spiegels die Laserstrahlenpulse nicht auf der Drehachse des zweiten Spiegels auftreffen, sondern abseits von dieser. Hierbei kann an Techniken zur Erzeugung eines 3-dimensionalen Scans aus dem Stand der Technik zurückgegriffen werden.

Bei einer bevorzugten Ausführungsform wird als Laserquelle ein Mikrochip-Laser verwendet, also umfasst die Laserquelle als abstrahlendes Medium einen elektromagnetische Strahlung, insbesondere Infrarotlicht, Licht oder Ultraviolettstrahlung, abgebenden Mikrochip. Bevorzugt weist diese elektromagnetische Strahlung, welche der Mikrochip im Betrieb abgibt, eine Wellenlänge aus dem Intervall von 300 und 400 nm auf, bevorzugt von 355 nm. Wie aus der DE 10 2007 028 042 B3 bekannt, wird zur Laserbearbeitung eines Patientenauges eine Wellenlänge aus dem Intervall von 300 bis 1000 nm ausgewählt und hierbei eine Pulslänge aus dem Intervall von 300 ps bis 20 ns gewählt. Die Bestrahlungstärke wird hierbei so gewählt, dass Plasmabildung ohne Plasmaleuchten in dem Patientenauge auftritt. Bei einem Mikrochip-Laser, also einem Laser, bei dem ein Mikrochip selbst das Licht emmitiert, sind Halbleiterschichten auf einem Substrat bereitgestellt, die eine lichtemittierende Vorrichtung bilden, z. B. eine Laserdiode. Durch den Schichtenaufbau ist hierbei in der Regel gleichzeitig ein Resonator bereitgestellt. Bei Verwendung eines Mikrochip-Lasers, der eine Wellenlänge von z. B. 355 nm abgibt, lässt sich das Verfahren aus der DE 10 2007 028 042 B3 besonders unaufwändig durchführen.

Die Teleskopanordnung weist in Kombination mit diesem Mikrochip-Laser den Vorteil auf, dass trotz der bei einem Mikrochip gegebenen kleinen Abstrahlfläche eine Abbildung auf einen relativ kleinflächigen Lichtfleck ermöglicht ist. Die Zwischenbildfläche kann hierbei größer sein als die Abstrahlfläche des Mikrochips.

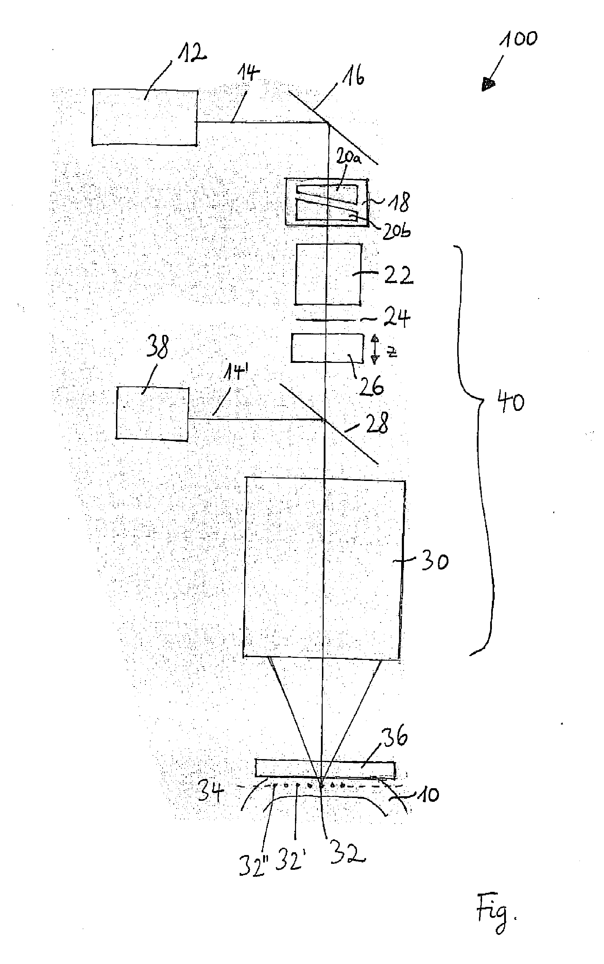

Nachfolgend wird eine bevorzugte Ausführungsform der Erfindung unter Bezug auf die Zeichnung beschrieben, die schematisch die Elemente einer erfindungsgemäßen Vorrichtung zeigt.

Eine im Ganzen mit 100 bezeichnete Vorrichtung zum Behandeln eines Patientenauges 10 umfasst eine Laserquelle 12, die Laserstrahlenpulse 14 abgibt. Diese werden über einen Ablenkspiegel 16 einer Einheit 18 mit zwei Risley-Prismen 20a, 20b zugeführt. Die Risley-Prismen 20a und 20b können unabhängig voneinander gedreht werden. Jede Drehung bewirkt ein Ablenken der Laserstrahlenpulse. Durch Kombination jeweiliger Drehwinkel der einzelnen Risley-Prismen 20a und 20b kann ein zweidimensionales Feld abgetastet werden.

Der Einheit 18 ist ein so genanntes F-Theta-Objektiv 22 nachgeordnet. Das F-Theta-Objektiv bildet ein Objekt auf eine Zwischenbildebene 24 ab. Vorliegend werden durch die Risley-Prismen 20a, 20b unterschiedlich abgelenkte Laserstrahlenpulse 14 auf unterschiedliche Stellen der Zwischenbildebene 24 abgelenkt. Kennzeichen des F-Theta-Objektivs 22 ist, dass die Bildhöhe proportional zum Einfallswinkel ist, der vorliegend dem durch die Risley-Prismen 20a, 20b erzeugten Auslenkwinkel entspricht, welcher auf den in der Figur gezeigten ablenkungsfreien geradlinigen Verlauf bezogen definiert ist.

Der Zwischenbildebene 24 nachgeordnet ist eine Linseneinheit 26 aus zumindest einer Linse, die in der Ausbreitungsrichtung der Laserstrahlenpulse (z-Richtung) verschieblich ist. Über einen teildurchlässigen Spiegel 28 gelangen die Laserstrahlenpulse aus dem optischen Element 26 zu einem Fokussierobjektiv 30, das die Laserstrahlenpulse auf einen Punkt 32 in der Hornhaut des Patientenauges 10 fokussiert. Durch Verschiebung der Linse in dem Linsenelement 26 längs der Ausbreitungsrichtung der Laserstrahlenpulse, der z-Richtung, lässt sich festlegen, wie tief der Punkt 32 in der Hornhaut 10 des Patientenauges sitzt, wie weit also eine Ebene 34, in der dieser Punkt liegt und welche parallel zur Zwischenbildebene ist, von der Zwischenbildebene 24 beabstandet ist.

Das Patientenauge 10 ist durch eine Applanierungsplatte 36 hierbei vorliegend vorne plattgedrückt. Punkte 32' und 32" in der Brennebene 34 werden von Laserstrahlenpulsen getroffen, die durch die Risley-Prismen 20a, 20b ausgelenkt sind. Die Risley-Prismen 20a, 20b bestimmen somit unmittelbar den Punkt des Auftreffens der Laserstrahlenpulse in dem Patientenauge 10 in der Brennebene 34.

Von dem teildurchlässigen Spiegel 28 wird ein Bild des Patientenauges, symbolisiert durch den Strahl 14', zu einer Kamera 38 geleitet, so dass das Patientenauge während der Behandlung beobachtbar ist.

Die Gesamtheit aus F-Theta-Objektiven 22, optischer Einheit 26 und Fokussierobjektiv 30 bildet eine im Ganzen mit 40 bezeichnete Teleskopanordnung. Das F-Theta-Objektiv 22 bildet hierbei den ersten Teil der Teleskopanordnung, die ein Zwischenbild 24 erzeugt, und die optische Einheit 26 bildet mit dem Fokussierobjektiv 30 den zweiten Teil der Teleskopanordnung 40. Die Zwischenbildebene 24 wird auf eine Brennebene 34 abgebildet. Dadurch ist es möglich, die Laserstrahlenpulse 14 stark fokussiert in das Patientenauge 10 zu applizieren. Die Größe eines Brennflecks 32 kann bis hinunter zu 1 bis 2 µm betragen.

Die Laserquelle 12 ist vorliegend als Mikrochip-Laser ausgebildet, der Licht mit einer Wellenlänge von 355 nm abgibt. Mit der Vorrichtung 100 ist insbesondere das Verfahren aus der DE 10 2007 028 042 B3 umsetzbar. Die Teleskopanordnung 40 gleicht hierbei den Nachteil mehr als aus, dass eine Mikrochip-Laserquelle 12 nur kleinflächig abstrahlt.

## Patentansprüche

1. Vorrichtung (100) zum Behandeln eines Auges (10) mit Laserstrahlung (14), mit einer Laserquelle (12) zur Abgabe von Laserstrahlenpulsen (14), **gekennzeichnet durch**
eine Teleskopanordnung (40), von der ein erster Teil (22) eine Abbildung der Laserstrahlenpulse (14) auf Punkte einer Zwischenbildfläche (24) erzeugt, und wobei der Zwischenbildfläche (24) im Strahlengang ein zweiter Teil (26, 30) der Teleskopanordnung (40) nachgeordnet ist, der die Zwischenbildfläche (24) auf Punkte einer Brennfläche (34) abbildet.

2. Vorrichtung (100) nach Anspruch 1,
**dadurch gekennzeichnet, dass**
der erste Teil der Teleskopanordnung (40) ein F-Theta-Objektiv (22) umfasst.

3. Vorrichtung (100) nach Anspruch 1 oder 2,
**dadurch gekennzeichnet, dass**
der zweite Teil der Teleskopanordnung (40) eine Linse (26) aufweist, die verschieblich ist, um so den Abstand der Brennfläche (34) von der Zwischenbildfläche (24) zu ändern.

4. Vorrichtung (100) nach einem der vorhergehenden Ansprüche,
**dadurch gekennzeichnet, dass**
zwischen der Laserquelle (12) und der Teleskopanordnung (40) Mittel (18) zum Ablenken der Laserstrahlenpulse angeordnet sind, damit unterschiedlich durch diese Mittel (18) abgelenkte Laserstrahlenpulse auf unterschiedliche Punkte (32, 32', 32") der Brennfläche (34) auftreffen.

5. Vorrichtung (100) nach Anspruch 4,
**dadurch gekennzeichnet, dass**
die Mittel zum Ablenken (18) zwei Risley-Prismen (20a, 20b) umfassen.

6. Vorrichtung nach Anspruch 4,
**dadurch gekennzeichnet, dass**
die Mittel zum Ablenken drei schwenkbare Spiegel umfassen.

7. Vorrichtung (100) nach einem der vorherigen Ansprüche,
**dadurch gekennzeichnet, dass**
die Laserquelle als das die elektromagnetische Strahlung für den Laser, insbesondere Infrarotlicht, Licht oder Ultraviolettstrahlung, abgebende Medium einen Mikrochip umfasst.

8. Vorrichtung (100) nach Anspruch 7,
**dadurch gekennzeichnet, dass**
die Laserquelle im Betrieb Laserstrahlenpulse (14) mit einer Wellenlänge aus dem Intervall von 300 bis 400 nm und bevorzugt von 355 nm abgibt.
